# FASCICULE DE BREVET EUROPEEN

(11) **EP 3 162 886 B1**
(45) Date de publication et mention de la délivrance du brevet: **01.01.2020**
(21) Numéro de dépôt: 16196122.2
(22) Date de dépôt: 27.10.2016
(51) Int. Cl.: C12M 1/42

(54) **DISPOSITIF DE TRAITEMENT D'AU MOINS UNE CELLULE BIOLOGIQUE, NOTAMMENT EN VUE D'UNE DELIVRANCE INTRACELLULAIRE**
VORRICHTUNG ZUR BEHANDLUNG MINDESTENS EINER BIOLOGISCHEN ZELLE, INSBESONDERE IM HINBLICK AUF EINE INTRAZELLULÄRE ZUFUHR
DEVICE FOR TREATING AT LEAST ONE BIOLOGICAL CELL, ESPECIALLY FOR INTRACELLULAR DELIVERY

(30) Priorité: 30.10.2015 FR 1560415
(43) Date de publication de la demande: 03.05.2017
(73) Titulaire: COMMISSARIAT À L'ÉNERGIE ATOMIQUE ET AUX ÉNERGIES ALTERNATIVES, 75015 Paris (FR); Institut National de la Santé et de la Recherche Médicale, 75013 Paris (FR)
(72) Inventeur: CASSET, Fabrice, 38570 Tencin (FR); MILLET, Arnaud, 38340 Voreppe (FR)
(74) Mandataire: Brevalex

(56) Documents cités:
- WO-A1-2013/185818
- WO-A1-2015/148842
- AU-A1- 2012 201 290
- US-A- 5 217 899
- US-A1- 2004 235 153
- US-A1- 2007 025 919
- US-A1- 2012 100 602
- US-A1- 2015 004 077

## Description

### DOMAINE TECHNIQUE ET ÉTAT DE LA TECHNIQUE ANTÉRIEURE

La présente invention se rapporte à un dispositif de traitement d'au moins une cellule biologique en vue d'une étape de délivrance intracellulaire, tel que défini dans les revendications.

Dans le domaine de la biomédecine on cherche à pouvoir introduire dans des cellules biologiques, par exemple des macromolécules, soit dans un but thérapeutique soit dans le cadre d'études. Ce procédé est désigné la délivrance intracellulaire.

Les macromolécules peuvent être de l'ADN ou des petits ARN interférents désignés siRNA, ou des molécules inorganiques comme des quantum dots ou des nanoparticules utilisés dans une visée thérapeutique, par exemple dans le traitement de cancers ou bien pour réaliser un marquage intracellulaire.

La membrane plasmique cellulaire est en grande partie imperméable aux macromolécules et des techniques ont été développées pour permettre cette délivrance intracellulaire.

Certaines techniques de délivrance intracellulaire de macromolécule reposent sur l'utilisation de nanoparticules polymériques, de liposomes, ou d'une modification de la molécule cible par l'ajout de peptide de pénétration par exemple. Ces techniques sont très dépendantes du type cellulaire et sont peu adaptées aux matériaux structurellement hétérogènes comme les protéines. En outre, ces techniques reposent sur la voie endosomale dont l'efficacité est inconstante. L'utilisation de vecteurs viraux pose le problème du non contrôle de l'insertion chromosomique et ne permettent que la délivrance d'ADN ou ARN.

Il existe également des techniques physiques de type électroporation par champ électrique ou sonoporation qui provoque l'apparition de pores dans la membrane plasmique par application d'un champ électrique. De telles techniques ont pour inconvénient d'avoir une mortalité cellulaire souvent élevée et peuvent détériorer les quantums dots par exemple.

Il existe également la micro-injection qui est utilisée en transgénèse, mais celles-ci ne permet pas de traiter un grand nombre de cellules.

Le document « A Vector-free microfluidic platform for intracellular delivery », A. Sharei and Al, PNAS, February 5, 2013, vol.110, no6, pages 2082-2087 décrit une technique visant à générer des pores dans la membrane plasmique de la cellule. Pour cela, les cellules sont en suspension dans un liquide et le dispositif comporte une ou plus zone de rétrécissement à travers lesquelles les cellules sont forcées à passer. Le diamètre des zones de rétrécissement sont tels qu'ils appliquent des contraintes de cisaillement et de compression aux cellules, ce qui provoque l'apparition de pores provisoires dans la membrane. L'ouverture de ces pores permet d'introduire des éléments dans les cellules. Ces pores se referment ensuite.

Cette technique est intéressante cependant elle ne peut s'appliquer directement aux cellules adhérentes. Il faudrait au préalable leur appliquer principalement un traitement enzymatique ce qui modifierait leur physiologie. En outre, les contraintes appliquées aux cellules et le temps pendant lequel ces contraintes sont appliquées, ne sont pas maîtrisés

### EXPOSÉ DE L'INVENTION

C'est par conséquent un but de la présente invention d'offrir un dispositif de traitement d'au moins une cellule, par exemple en vue d'une délivrance intracellulaire, ne présentant pas les inconvénients des dispositifs de l'état de la technique.

Le but énoncé ci-dessus est atteint par un dispositif mettant en œuvre un support de traitement sur lequel des cellules biologiques sont destinées à adhérer, et des moyens commandables pour déformer le support de traitement de sorte à appliquer une contrainte mécaniques aux cellules, et en particulier à leur membrane plasmique. Cette contrainte peut être suffisante pour provoquer l'apparition d'au moins un pore transitoire dans la membrane plasmique.

On entend par « pore transitoire » dans la présente demande, une ouverture se formant dans la membrane plasmique par application d'une contrainte mécanique, de cette membrane, le pore se refermant de lui-même après un certain temps, à l'inverse d'un pore définitif résultant d'une disruption de la cellule.

Grâce à l'invention, la déformation de la cellule est maîtrisée, puisque le type de contrainte et le niveau de contrainte appliqué aux cellules et le temps d'application de cette contrainte sont maîtrisés en commandant les moyens de déformation.

Dans un exemple très avantageux, la formation de pores est maîtrisée.

Le dispositif selon l'invention permet une délivrance cellulaire non dépendante de la voie d'endocytose.

Par exemple, les moyens commandables peuvent être du type actionneur piézoélectrique, électrostatique, magnétique ou thermique.

Grâce au dispositif selon l'invention, le taux de mortalité des cellules biologiques traitées est sensiblement réduit par rapport aux techniques de l'état de la technique, telle que l'électroporation. Il peut donc être utilisé à des visées thérapeutiques.

Le dispositif selon l'invention permet le maintien dans le temps d'une contrainte sur la cellule par exemple pour l'étude de la réponse mécanique par exemple par microscopie par force atomique. Ce type d'étude se révèle d'une grande importance notamment dans l'étude des cellules cancéreuses.

Ce dispositif est en outre particulièrement adapté au traitement des cellules telles que des macrophages humains, des cellules dendritiques humaines puisque celles-ci sont naturellement adhérentes. Le traitement de ces cellules est très intéressant car la modification de ces cellules par délivrance intracellulaire est un enjeu majeur dans le domaine de l'immunothérapie, d'autant plus que la délivrance intracellulaire pour ces cellules est particulièrement difficile avec les techniques traditionnelles.

De manière très avantageuse, le dispositif est un dispositif microélectromécanique (MEMS pour MicroelectromechanicalSystems) et ou nanoélectromécanique (NEMS pour NanoelectromechanicalSystems), ce qui le rend encore plus adapté au traitement de cellules biologiques.

La présente invention a alors pour objet un dispositif microfluidique de délivrance à au moins une cellule biologique comportant :
- au moins un support de traitement comprenant une surface d'accueil permettant l'adhérence de ladite au moins une cellule biologique,
   des moyens d'alimentation en une solution comportant au moins une cellule biologique à traiter,
- des moyens d'évacuation de ladite solution,
- des moyens pour alimenter en une solution contenant des éléments à introduire dans la cellule
- des moyens configurés pour appliquer une contrainte à la cellule (C) pour provoquer l'apparition d'au moins un pore transitoire dans la membrane de la cellule (C) par déformation du support de traitement, lesdits moyens comportant au moins un actionneur apte déformer ledit support afin d'appliquer une contrainte à ladite au moins une cellule, et des moyens de commande dudit au moins un actionneur de sorte que l'actionneur déforme le support de traitement selon une amplitude de déformation donnée et pendant une durée de traitement donnée de sorte à appliquer ladite contrainte à la cellule pendant ladite durée de traitement.

Dans la présente demande, on entend par « traitement » le fait de modifier la cellule biologique par déformation de celle-ci lors de l'application d'une contrainte mécanique. Cette déformation permet de générer au moins un pore dans la cellule et/ou déterminer la réponse mécanique de la cellule à la contrainte notamment pour la caractériser et en particulier discriminer des cellules telles que des cellules cancéreuses.

L'amplitude de déformation peut permettre de générer au moins un pore transitoire dans une membrane de la cellule biologique de sorte à traiter ladite cellule et/ou l'amplitude de déformation peut permettre de déterminer la réponse mécanique de la cellule à la contrainte de sorte à la caractériser.

Dans un exemple de réalisation, le support de traitement est contenu principalement dans plan moyen et l'actionneur est apte à déformer le support de traitement selon une direction hors plan.

Dans un autre exemple de réalisation, le support de traitement est contenu principalement dans plan moyen et l'actionneur est apte à déformer le support de traitement principalement dans ledit plan moyen.

Le dispositif microfluidique de délivrance peut comporter un actionneur ou plusieurs actionneurs disposés de sorte à appliquer une déformation axisymétrique au support de traitement.

En variante, le dispositif microfluidique de délivrance peut comporter un actionneur ou plusieurs actionneurs disposés de sorte à appliquer une déformation cylindrique au support de traitement.

Avantageusement, tout ou partie de la surface du support de traitement est fonctionnalisée de sorte à modifier la force d'adhérence du ou des types de cellules biologiques à traiter par rapport à la surface non fonctionnalisée.

L'actionneur peut être un actionneur piézoélectrique ou ferroélectrique ou thermique ou un actionneur électrostatique ou magnétique
Le dispositif microfluidique de délivrance peut par exemple comporter un support de traitement en matériau souple et le au moins actionneur peut être tel qu'il peut appliquer un effort de traction à une zone de bord du support de traitement dans le plan moyen. Avantageusement le dispositif comporte au moins deux actionneurs appliquant un effort de traction en sens opposés à deux bords opposés du support de traitement.

Dans le cas où les actionneurs sont des actionneurs électrostatiques à peignes interdigités, chaque actionneur peut comporter un peigne mobile et un peigne fixe définissant entre eux un entrefer, le support de traitement étant suspendu entre les deux peignes mobiles, la variation dudit entrefer appliquant une contrainte de traction audit support de traitement.

Le support de traitement peut être en un matériau souple et l'actionneur peut comporter au moins une poutre encastrée-libre et un matériau piézoélectrique ou ferroélectrique solidaire de la poutre, de sorte que la déformation du matériau piézoélectrique ou ferroélectrique provoque une déformation hors plan de la poutre et du support de traitement.

Dans un autre exemple de réalisation, le dispositif microfluidique de délivrance peut comporter plusieurs actionneurs répartis au niveau d'un bord extérieur du support de traitement.

Le dispositif microfluidique de délivrance peut par exemple être un dispositif MEMS et/ou NEMS

La présente invention a également pour objet un procédé de délivrance cellulaire mettant en œuvre le dispositif microfluidique de délivrance cellulaire selon l'invention, comportant les étapes:
- mise en contact de la solution contenant la au moins une cellule biologique à traiter avec le support de traitement,
- adhérence de ladite au moins une cellule au support de traitement,
- alimentation en solution contenant les éléments à introduire dans la cellule,
- application d'une contrainte à la cellule pour provoquer l'apparition d'au moins un pore transitoire dans la membrane de la cellule par déformation du support de traitement,
- arrêt de l'application de la contrainte,
- fermeture du au moins un pore transitoire,
- évacuation de la cellule.

Dans un mode de fonctionnement, le support de traitement est déformé dans son plan, et dans un autre mode de fonctionnement le support de traitement est déformé selon une direction hors plan.

### BRÈVE DESCRIPTION DES DESSINS

La présente invention sera mieux comprise sur la base de la description qui va suivre et des dessins en annexe sur lesquels:
- la figure 1 est une représentation schématique générale d'un exemple de réalisation d'un dispositif de traitement selon l'invention,
- la figure 2 est une vue en coupe d'un exemple d'un dispositif de traitement muni de moyens de déformation commandables de type piézoélectrique pouvant être mis en œuvre dans le dispositif de la figure 1,
- la figure 3A est une vue de dessus représenté schématiquement du support de traitement mis en œuvre dans le dispositif de la figure 2,
- les figures 3B et 3C sont des vues en coupe le long du plan A-A du support de traitement de la figure 3A dans deux états différents,
- la figure 4 est une vue en coupe schématique d'un exemple d'un dispositif de traitement muni de moyens de déformation commandables de type électrostatique pouvant être mis en œuvre dans le dispositif de la figure 1,
- la figure 5A est une vue de dessus d'un autre exemple de réalisation d'un support de traitement muni de moyens de déformation commandables de type piézoélectrique,
- les figures 5B et 5C des vues en coupe le long du plan B-B d'un dispositif de traitement mettant en œuvre le support de traitement de la figure 5A intégré dans un dispositif de traitement dans un état n'appliquant pas de déformation et un état appliquant une déformation respectivement,
- les figures 6A et 6B sont de vues de dessus et en coupe transversale respectivement d'un exemple de réalisation d'un support de traitement apte à appliquer une déformation cylindrique à une cellule,
- les figures 7A et 7B sont de vues de dessus et en coupe transversale respectivement d'un autre exemple de réalisation d'un support de traitement apte à appliquer une déformation cylindrique à une cellule biologique,
- la figure 8 est une représentation schématique d'une cellule subissant une déformation cylindrique,
- les figures 9A et 9B sont de vues de dessus et en coupe transversale respectivement d'un support de traitement à actionnement électrostatiques apte à appliquer une déformation uniaxiale à la cellule biologique,
- les figures 10A à 10S sont des représentations schématiques des éléments obtenus au cours des différentes étapes d'un exemple de procédé de réalisation d'un dispositif de traitement,
- les figures 10N' à 10R' sont des représentations schématiques d'éléments obtenus au cours des différentes étapes selon une variante d'un procédé de réalisation d'un dispositif de traitement.

### EXPOSÉ DÉTAILLÉ DE MODES DE RÉALISATION PARTICULIERS

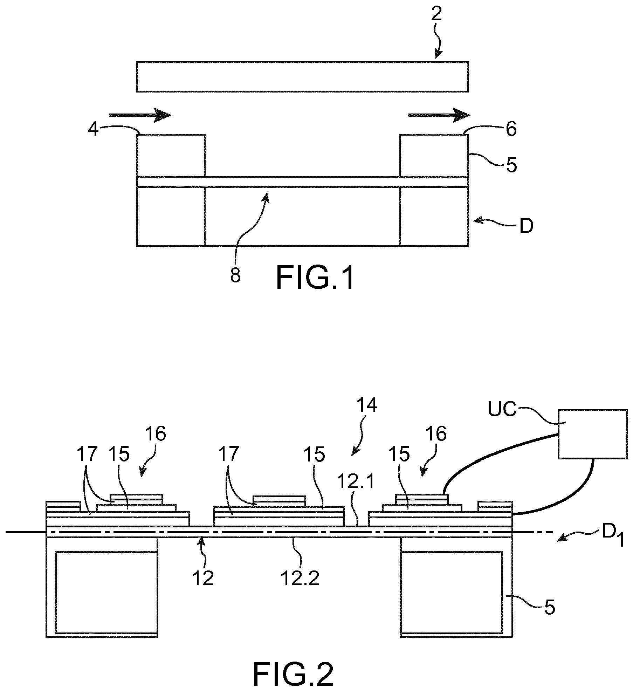

Sur la figure 1, on peut voir une représentation schématique d'un exemple d'un dispositif de traitement. Le dispositif de traitement permet d'appliquer une contrainte à la cellule, par exemple pour générer un ou des pores dans la membrane de la cellule, ces pores pouvant permettre par exemple la délivrance d'éléments donnés à la cellule, mais pas exclusivement.

Dans l'exemple représenté, le dispositif de traitement comporte un boîtier 2 muni d'une entrée d'alimentation fluidique 4 réalisée dans une paroi latérale 5 du boîtier et d'une sortie d'évacuation fluide 6 réalisée dans la paroi latérale 5 du boîtier et un fond 8. L'entrée d'alimentation 4 et la sortie d'évacuation 6 sont disposées par rapport au fond 8 de sorte que le flux de fluide s'écoule sur le fond de l'entrée 4 vers la sortie 6.

Le fond 8 est formé par un dispositif apte à déformer des cellules biologiques venant adhérer sur celui-ci.

Sur la figure 2, on peut voir un exemple de réalisation du dispositif D1 apte à déformer des cellules biologiques venant adhérer sur celui-ci mettant en œuvre des moyens piézoélectriques.

Le dispositif D1 comporte un support de traitement 12, dans l'exemple représenté, formé par une plaque fine suspendue à la paroi latérale du boîtier et apte à se déformée élastiquement. Le support de traitement forme dans cet exemple le fond boîtier. Le dispositif D1 comporte également au moins un actionneur apte à déformer le support de traitement 12 dans une direction hors plan. Dans l'exemple représenté, le dispositif comporte deux actionneurs 14, 16 mettant en œuvre un matériau ferroélectrique, par exemple du PZT, l'un des actionneurs sert au déplacement de la membrane vers le haut et l'autre actionneur sert au déplacement de la membrane vers le bas. Dans le cas où un matériau piézoélectrique est mis en œuvre, un seul actionneur est suffisant pour assurer les déplacements vers le haut et vers le bas, puisqu'un matériau piézoélectrique se dilate et se contracte suivant le signe de la tension appliquée.

Le support de traitement 12 a une forme circulaire, l'actionneur 14 est un actionneur ferroélectrique et est disposé sensiblement au centre du support de traitement 12 sur une face 12.1 opposée à celle 12.2 située dans le boîtier et destinée à entrer en contact avec le fluide. L'actionneur 16 est également un actionneur ferroélectrique de forme annulaire et bordant le bord extérieur de la surface extérieure 12.1 de la membrane 12.

Les actionneurs 14 et 16 sont reliés à une unité de commande UC permettant de commander les actionneurs pour qu'ils provoquent une déformation du support de traitement selon une amplitude donnée et pendant un temps donné.

La surface intérieure 12.2 est telle qu'elle permet l'adhérence des cellules que l'on souhaite traiter. La surface 12.2 est par exemple en verre, en silicium ou tout matériau semi-conducteur.

De manière avantageuse, la surface du support de traitement 12 peut être fonctionnalisée pour délimiter une ou des zones préférées d'adhérence du ou des cellules.

Par exemple, la fonctionnalisation de la membrane est obtenue par un dépôt (ou « coating » en terminologie anglo-saxonne) par exemple de molécules de la matrice extracellulaire, par exemple des molécules de fibronectine ou de laminine, ou par fixation chimique d'anticorps spécifique du type cellulaire étudié. Le dépôt de fonctionnalisation est par exemple un dépôt chimique qui est réalisé en exposant la surface à traiter à une solution contenant les molécules à déposer et attendre l'adsorption de ces molécules sur la surface de la membrane.

En variante, on peut former des zones localisées de matériau sur le support de traitement, par exemple de type nitrure ou oxyde, favorisant l'adhérence des cellules lors du procédé de réalisation microélectronique de la membrane. Par exemple, une couche de ce matériau est formée sur la couche destinée à former la membrane et les zones d'adhérence sont déterminées par photolithographie et gravure.

Il sera compris que l'on peut déposer un matériau qui, au contraire réduit, voir empêche l'adhérence des cellules, pour délimiter des zones où l'adhérence est défavorisée, les cellules adhérant entre ces zones.

On peut prévoir en plus ou à la place de traiter les cellules pour favoriser leur adhérence à un certain matériau par exemple en fixant sur du support de traitement des éléments qui eux offrent une bonne adhérence avec le matériau du support de traitement.

En variante, on pourrait envisager que ce soit le côté du dispositif portant le ou les actionneurs qui permette l'adhérence cellules; des moyens pour isoler électriquement les actionneurs du fluide contenant les cellules seraient alors prévus, par exemple une couche isolante électrique.

Cette fonctionnalisation s'appliquent à tous les exemples qui sont décrits dans la suite de la description.

Les actionneurs 14, 16 comportent un matériau ferroélectrique ou piézoélectrique 15 et deux électrodes 17 de part et d'autres du matériau 15 permettant d'appliquer à ce matériau 15 un champ électrique sur commande, qui provoque une déformation dans le plan du matériau et donc une déformation hors plan de la membrane dont il est solidaire, par effet bilame.

L'optimisation de tels actionneurs sont décrits dans le document F. Casset et al, « Piezoelectric membrane actuator design », 12th Int. Conf.On Thermal, Mechanical and Multiphysics Simulation and Experiments in Microelectronics and Microsystems (IEEE Eurosime), 18-20 april2011, pp. 1-5*.*

En variante, la membrane pourrait avoir une toute autre forme, par exemple être d'une forme carrée ou rectangulaire.

En variante, un seul actionneur pourrait être mis en œuvre.

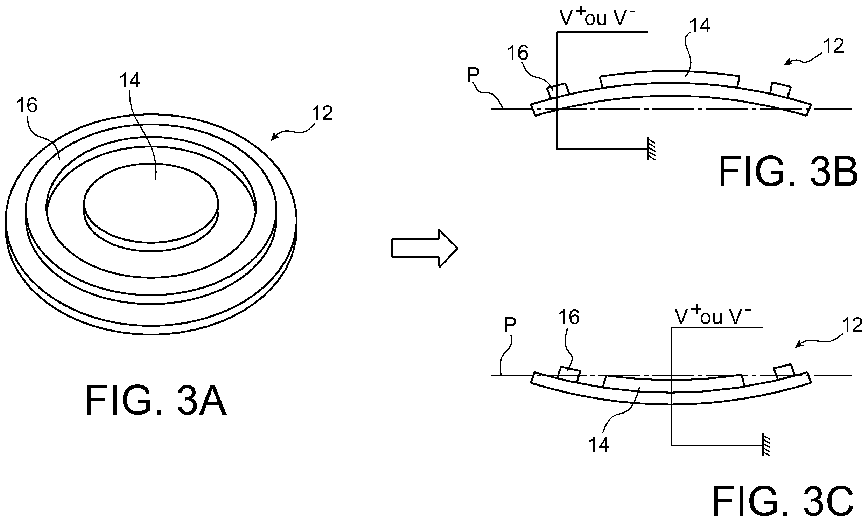

Sur les figures 3A à 3C, on peut voir le support de traitement de la figure 2 vu de dessus et le dispositif dans deux états de déformation.

Sur les figures 3A à 3C, seule la membrane est représentée avec les actionneurs.

Dans l'exemple représenté, l'application d'une tension sur l'actionneur 16 provoque un déplacement du support de traitement 12 vers le haut. L'application d'une tension sur l'actionneur 14 provoque un déplacement de la membrane 12 vers le bas.

Dans un autre exemple de réalisation, les actionneurs sont réalisés à partir de matériaux piézoélectriques comme par exemple AIN, ZnO... Un champ électrique hors plan; dans le même sens que la polarisation dans le matériau va induire une extension dans le plan du matériau piézoélectrique. Inversement, un champ électrique hors plan, inversé par rapport à la polarisation du matériau va induire une contraction dans le plan du matériau piézoélectrique. Ainsi les déplacements vers le haut et le bas peuvent être obtenus en utilisant un seul actionneur. L'amplitude du déplacement de la membrane est proportionnelle à la tension appliquée aux bornes des actionneurs.

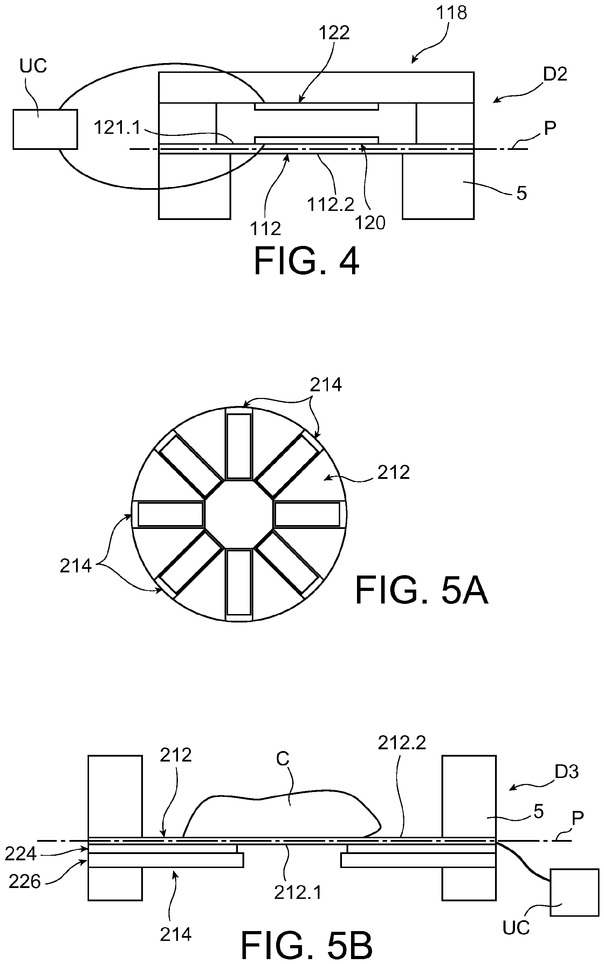

Sur la figure 4, on peut voir un autre exemple de dispositif D2 apte à déformer des cellules biologiques venant adhérer sur le support de traitement mettant en œuvre des moyens électrostatiques.

Le dispositif comporte une partie fixe 118 et un support de traitement suspendu 112 sur la partie fixe 118. Les moyens électrostatiques permettant de déformer le support de traitement 112 comporte au moins une première électrode 120 portée par une face 112.1 support de traitement opposée à celle 112.2 sur laquelle les cellules sont destinées à adhérer et au moins une deuxième électrode 122 sur la partie fixe 118 en regard de la première électrode 120. Les première 120 et deuxième 122 électrodes sont connectées à une source de tension ou de courant (non représentée) qui est commandée par l'unité de commande UC.

Le support de traitement est par exemple en polysilicium ou en tout autre matériau semi-conducteur.

Lorsque une différence de tension est appliquée entre les électrodes 120 et 122, un champ électrique apparaît entre les électrodes et provoquent une attraction ou une répulsion de la première électrode par rapport à la deuxième électrode 120 et donc un déplacement du support de traitement 112 qui du fait de son ancrage sur la partie fixe, se déforme.

Selon un autre exemple de réalisation non représenté, le dispositif apte à déformer des cellules biologiques venant adhérer sur celui-ci peut comporter un ou des actionneurs thermiques.

Le fonctionnement d'un actionneur thermique est similaire à celui d'un actionneur piézoélectrique. Un courant traverse l'actionneur thermique solidaire de la membrane, le courant provoque une augmentation de la température de l'actionneur par effet Joule. Cette augmentation de température induit une dilatation thermique provoquant un couple mécanique déformant la membrane. Dans cet exemple de réalisation, on peut prévoir avantageusement des moyens pour maîtriser la température du milieu liquide des cellules pour éviter une augmentation de la température qui pourrait être préjudiciable aux cellules. Par exemple on peut prévoir de refroidir de manière contrôlée le milieu liquide.

Selon un autre exemple de réalisation non représenté, le dispositif apte à déformer des cellules biologiques venant adhérer sur celui-ci peut comporter un ou des actionneurs magnétiques. Par rapport à un actionneur électrostatique, l'électrode solidaire de la membrane est remplacée par un matériau magnétique et des moyens aptes à générer un champ magnétique pour attirer ou repousser le matériau magnétique sont prévus. Le support de traitement est alors déformé suivant le champ magnétique généré.

Le fonctionnement du dispositif de la figure 2 va maintenant être décrit.

Lorsqu'une ou des cellules ont adhérées sur le support de traitement, celui-ci est déformé par les moyens de déformation. Pour cela une tension continue est appliquée aux moyens de déformation, par exemple aux électrodes 17 des actionneurs piézoélectriques. La ou les cellules disposées sur le support sont alors également déformées. La déformation peut être suffisante pour provoquer la génération d'un ou de plusieurs pores dans la membrane plasmique. La valeur de a tension appliquée aux électrodes a été déterminée pour provoquer une déformation de la cellule et l'apparition de pores mais évitant une disruption complète de la membrane. Alternativement la contrainte applique à la membrane déforme la cellule sans générer de pore.

Sur les figures 5A à 5C, on peut un autre exemple de réalisation d'un dispositif de traitement D3 offrant une déformation amplifiée par rapport au dispositif des figures 2 et 3A à 3C
Le dispositif comporte un support de traitement souple 212, par exemple en matériau synthétique, type polymère. On entend par « support souple » un support présentant une faible rigidité du fait de son faible module d'Young, compris entre quelques centaines de MPa à une dizaine de GPa. Par exemple le support est en polycarbonate, polyethylènenaphtalate...

Dans l'exemple représenté, le support de traitement 212 a la forme d'un disque et est encastré au niveau de sa périphérie extérieure entre la paroi 5 du dispositif confinant le milieu des cellules et les actionneurs. La face 212.2 du support de traitement qui forme la face supérieure du support de traitement dans la représentation de la figure 5A est celle sur laquelle les cellules vont adhérer. Le dispositif comporte également plusieurs actionneurs piézoélectriques 214 répartie sous le support de traitement comprenant chacun un élément 224 en matériau piézoélectrique encastré-libre disposée sous le support de traitement et en contact avec sa face inférieure 212.1 et des électrodes (non représentées) de part et d'autre de l'élément pour lui appliquer une tension de commande. Les éléments 224 sont encastrés par leur extrémité radialement extérieure et sont libres au niveau de leur extrémité radialement intérieure. Chaque actionneur 214 est associé à une poutre encastrée libre 226 superposée à l'élément 224 et solidaire en mouvement de celui-ci. La poutre 226 est telle qu'elle est déformable en flexion sous l'action de l'actionneur 214. Les poutres 226 sont encastrées par leur extrémité radialement extérieure et sont libres au niveau de leur extrémité radialement intérieure.

Dans l'exemple représenté, le dispositif comporte plusieurs ensembles actionneur-poutre répartis en étoile sous le support de traitement de manière uniforme. La mise en œuvre de poutres permet d'augmenter l'amplitude de déformation du support de traitement souple 212 du fait de leur rigidité réduite par rapport au support. Les poutres permettent ainsi d'augmenter la déformation de la membrane et donc les contraintes tangentielles appliquées dans le plan de la membrane

Le fonctionnement de ce dispositif est le suivant. En l'absence de tension appliquée aux actionneurs 214, le support de traitement 212 est sensiblement plan et la cellule C n'est pas déformée (figure 5B).

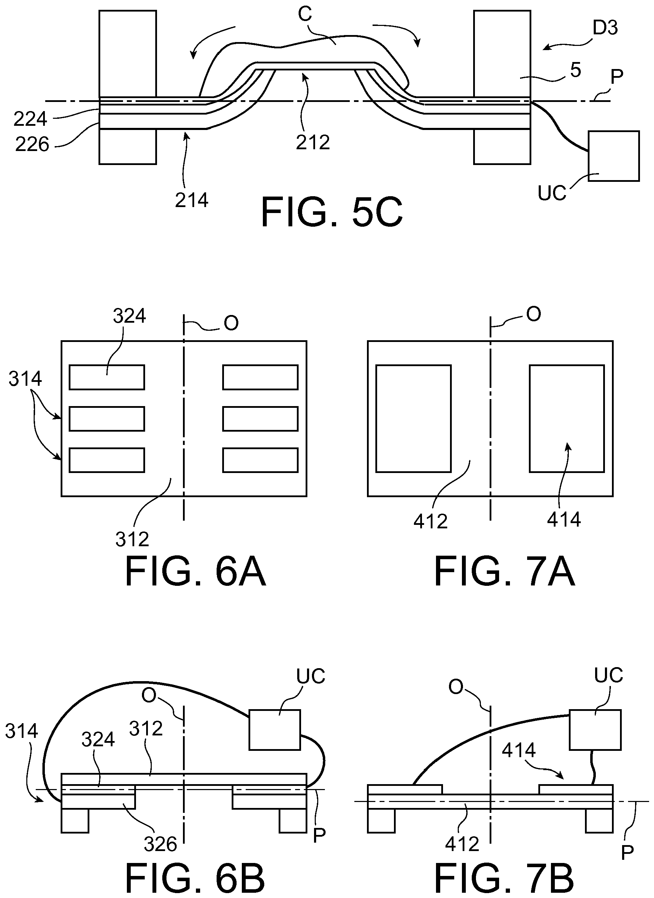

Lorsqu'une tension est appliquée à chacun des actionneurs 214, les éléments piézoélectriques224 se déforment, déformant les poutres 226 qui fléchissent provoquant la déformation du support de traitement 212et donc de la cellule C qui a adhérée à celui-ci (figure 5C).

Cette réalisation permet d'amplifier la déformation subie par la cellule C par rapport à celle appliquée par le dispositif de la figure 2.

Les supports de traitement et les moyens de déformation associés décrits en relation avec les figures 1 à 5C assurent une déformation sphérique de la cellule, i.e. une déformation axisymétrique, comme cela est schématisé sur la figure 5C.

Le dispositif de la figure 5A pourrait néanmoins être commandé pour appliquer une contrainte non axisymétrique, par exemple en activant un seul actionneur 214 ou en activant une paire d'actionneurs 214 alignés ou plusieurs paires d'actionneurs 214 alignés de sorte à appliquer une contrainte selon un ou plusieurs axes privilégiés.

Par ailleurs, un dispositif similaire à celui de la figure 5A mais ne comportant qu'un actionneur ou une ou des paires d'actionneurs ne sort pas du cadre de la présente invention.

En outre, on peut envisager d'appliquer à un ou certains actionneurs une tension positive et à un ou d'autres actionneurs une tension négative de sorte à provoque une déformation de la membrane des deux côtés du plan de la membrane au repos. En outre, on peut prévoir de ne pas appliquer la même tension à tous les actionneurs de sorte que des niveaux de déformation différents soient appliqués à la membrane.

Il sera compris que les actionneurs du dispositif de la figure 5A peuvent être remplacées en tout ou partie par des actionneurs thermiques, électrostatiques, magnétiques...

Sur les figures 6A à 7B, on peut voir un autre exemple de dispositif permettant d'appliquer une déformation cylindrique à la cellule, i.e. une déformation autour d'un axe.

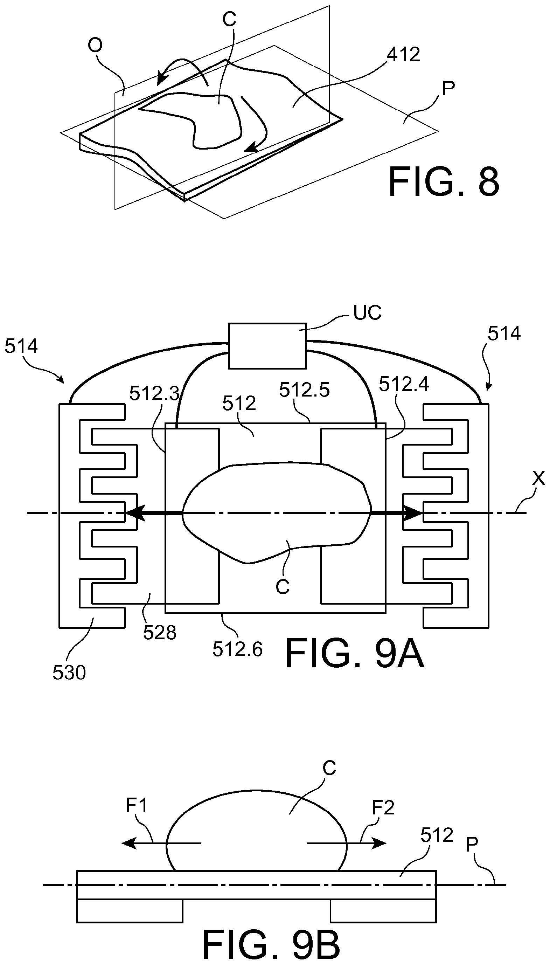

Le dispositif des figures 6A et 6B est du même type que celui de la figure 5A, il comporte des éléments piézoélectriques 324 et des poutres 326 encastrées libres sous un support de traitement en matériau souple 312, par exemple en matériau polymère. Dans l'exemple représenté le support de traitement 312 est de forme rectangulaire et les actionneurs 314 sont répartis de part et d'autre d'un plan de symétrie O support de traitement 312 orthogonal au plan médian de la membrane. Ainsi lorsqu'une même tension est appliquée aux actionneurs 314, le support de traitement voit sa zone centrale le long du plan de symétrie O se déplacer d'un côté ou de l'autre du plan médian comme cela est représenté sur la figure 8. Dans l'exemple représenté et de manière avantageuse, les actionneurs 314 sont répartis de manière symétrique de part et d'autre du plan O permettant d'appliquer une déformation cylindrique à la cellule.

En variante, on pourrait envisager que les actionneurs ne soient pas disposés de manière symétrique par rapport au plan O. On pourrait alors prévoir de n'actionner que les actionneurs positionnés symétriquement par rapport au plan O, si l'on veut appliquer une déformation cylindrique à la cellule.

Le dispositif des figures 7A et 7B est du même type que celui de la figure 2. Dans l'exemple représenté, le support de traitement 412 est de forme rectangulaire et les actionneurs 414 sont répartis de part et d'autre d'un plan de symétrie O du support de traitement 412 orthogonal au plan médian du support de traitement. Ainsi lorsqu'une même tension est appliquée aux actionneurs, le support de traitement412 voit sa zone centrale le long du plan de symétrie O se déplacer d'un côté ou de l'autre du plan médian comme cela est représenté sur la figure 8. Comme pour le dispositif des figures 6A et 6B, les actionneurs 414 sont répartis de manière symétrique de part et d'autre du plan O, mais ceci n'est en aucun cas limitatif, ils pourraient par exemple être disposés en quinconce ou de tout autre manière.

Sur la figure 9A et 9B, on peut voir un autre exemple de dispositif mettant en œuvre des actionneurs électrostatiques.

Le dispositif comporte un support de traitement souple 512, par exemple en matériau synthétique, tel qu'un polymère. Dans l'exemple représenté, le support de traitement 512 est de frome rectangulaire. Le dispositif comporte également deux actionneurs 514, disposés chacune au niveau de deux bords 512.3, 512.4 opposés de la membrane le long d'un axe X.

Les deux actionneurs sont de structures similaires, seulement l'un des actionneurs sera décrit en détail.

L'actionneur est du type à peignes interdigités. Il comporte un peigne fixe 530 et un peigne mobile 528 solidarisé au bord 512.3 du support de traitement 512.

Le fonctionnement du dispositif est le suivant, lorsqu'une tension est appliquée entre le peigne fixe 530 et le peigne mobile 528, le peigne mobile se rapproche du peigne fixe par attraction électrostatique. Le bord du support de traitement solidaire du peigne mobile est déplacée le long de l'axe X vers la gauche dans la représentation des figures 9A et 9B. Ce déplacement est symbolisé par la flèche F1. Lorsque les deux actionneurs sont alimentés et que les deux peignes mobiles s'éloignent de leur peigne fixe associée, le support de traitement est étiré selon les flèches F1 et F2 dans le plan P, et la cellule C adhérant au support de traitement 512 subit la même déformation. Ce dispositif permet d'appliquer une déformation uniaxiale à la cellule.

En variante, l'actionneur pourrait ne comporter qu'un peigne mobile interdigité avec un peigne fixe, le support serait alors ancré sur le peigne mobile sur un ancrage mécanique fixe. Le support serait alors étiré par déplacement du peigne mobile par rapport à l'ancrage mécanique. Dans cette variante, la contrainte applicable serait néanmoins deux fois plus faible que celle applicable avec le dispositif de la figure 9A.

En variante encore, on pourrait prévoir un seul actionneur. Un bord du support de traitement serait solidaire d'un peigne fixe et l'autre bord serait solidaire du peigne mobile. Lorsqu'une tension est appliquée entre les deux peignes, le peigne mobile s'écarte du peigne fixe emportant avec lui le bord qui lui est solidaire, appliquant une traction au support de traitement.

On peut envisager de réaliser un dispositif avec plusieurs paires d'actionneurs, ainsi des déformations selon plusieurs axes peuvent être appliquées. Par exemple, on peut prévoir de disposer une paire d'actionneur le long d'un axe perpendiculaire à l'axe X, les peignes mobiles étant solidaires des bords 512.5, 512.6. En actionnant les différentes paires d'actionneurs simultanément, une contrainte de traction axisymétrique peut être appliquée à la cellule.

En variante, les actionneurs électrostatiques pourraient être remplacés par des actionneurs piézoélectriques tels que ceux décrits dans le document Yoshida et al, « Fabrication and characterization of laterally-driven piezoelectric bimorph MEMS actuator with sol-gel-based high aspect ratio PZT structure », J. Micromech. Microeng. 23 (2013) 065014 *(llpp).De* tels actionneurs comportent des poutres encastrées libres déformables dans le plan, comprenant une âme en silicium perpendiculairement au plan du support de traitement et des couches de matériau piézoélectrique de part et d'autre de l'âme et s'étendant perpendiculairement au plan du support de traitement. Les extrémités libres des poutres sont solidaires d'un bord du support de traitement. En prévoyant de tels actionneurs au niveau de deux bords opposés du support de traitement, il est possible d'appliquer une traction au support de traitement et à la cellule qui adhère au support de traitement.

A titre d'illustration pratique, nous allons donner des valeurs de dimensionnement estimés du support de traitement et des tensions à appliquer pour former un pore dans la membrane plasmique de la cellule.

L'ordre de grandeur de la tension linéaire pour créer un pore dans une cellule biologique est de 1.10⁻¹¹j/m. Pour créer un pore de 100 nm de diamètre dans la membrane d'une cellule de 5 µm à 10 µm de diamètre, l'ordre de grandeur de l'énergie requise est de l'ordre de 1.10⁻¹⁸J.

Considérons le cas du dispositif de la figure 2 ou du dispositif des figures 6A à 7B dans lequel la déformation de la cellule est obtenue en imposant un rayon de courbure à symétrie sphérique ou symétrie cylindrique respectivement. Il a été estimé que l'ordre de grandeur du rayon de courbure du support de traitement était de 3 µm pour former un pore de 100 nm de diamètre ou de 10 µm pour former un pore de 10 nm de diamètre. Il s'agit de valeur haute car ces estimations concernent la formation de pores définitifs, i.e. qui ne se referment pas. Il est estimé que le rayon de courbure pour former des pores transitoires est de l'ordre de 10⁴ µm et que ce rayon de courbure peut être obtenu au moyen d'un support de traitement de 600 µm de diamètre, celle-ci présentant une déformation de l'ordre de 4,5 µm. En considérant un actionneur piézoélectrique, une tension de l'ordre de 50 V va permettre d'obtenir la déformation requise du support de traitement. Ces dimensions et valeur de tension sont compatibles avec les dispositifs microélectromécaniques et/ou nanoélectromécaniques.

Dans le cas où on déforme la cellule par étirement comme cela est le cas avec les dispositifs des figures 9A et 9B, il a été estimé que le déplacement à appliquer est de l'ordre du dixième de µm. A titre d'exemple, on peut obtenir environ 4 µm de déformation sous 10V pour une membrane de rayon 800 µm.

Les étapes d'utilisation d'un dispositif selon l'invention pour permettre la délivrance cellulaire à une cellule vont maintenant être décrites. Le cas d'une seule cellule va être décrit, mais il sera compris qu'en général plusieurs cellules sont traitées simultanément par le même dispositif en fonction de la taille du support de traitement.

Lors d'une première étape, la cellule que l'on veut traiter, i.e. à qui une délivrance cellulaire est à appliquer, est introduite dans le dispositif par l'entrée d'alimentation. La cellule est en général en suspension dans un milieu liquide. On utilise par exemple une pipette qui peut dispenser un volume de liquide et la quantité de cellule souhaités. En variante, un circuit microfluidique peut être connecté à l'entrée d'alimentation et assurer la circulation du fluide contenant les cellules entre un réservoir et le dispositif.

Lors d'une deuxième étape, la cellule adhère au support de traitement. Pour cela une phase de sédimentation a lieu lors de laquelle la cellule va venir se déposer par gravité sur le support de traitement. La cellule s'accroche alors à la membrane et adhère. La durée de cette étape peut être de l'ordre de plusieurs dizaines de minutes. Plus cette étape est longue, plus l'intensité de la force d'adhésion est grande. Le contrôle de la durée de cette étape permet donc de contrôler la force d'adhésion de la cellule sur la membrane.

Comme expliqué ci-dessus, la surface du support peut comporter des zones facilitant l'adhésion des cellules ou au contraire la rendant plus difficile. Dans ce cas la cellule adhère dans une zone où l'adhésion est favorisée ou au moins non détériorée.

En variante, il peut être envisagé d'utiliser un ou des pochoirs au-dessus de la surface du support, le ou les pochoirs étant réalisé(s) en un matériau qui ne favorise pas l'adhésion cellulaire et comporte(nt) des orifices aux endroits où l'on souhaite que les cellules se déposent. Le pochoir ou les pochoirs est ou sont ensuite retiré(s) à la fin de l'étape de sédimentation.

Lors d'une étape suivante, le milieu contenant les éléments à introduire dans la cellule est injecté dans le dispositif. Ce milieu est par exemple un milieu de culture contenant les macromolécules à introduire dans la cellule. Ce milieu peut être injecté au moyen d'une pipette ou d'un circuit fluidique, qui peut être le même que celui transportant le milieu contenant les cellules. Avantageusement, le milieu contenant les éléments à introduire dans la cellule sera injecté dans le dispositif de traitement préalablement à l'ouverture des pores.

Lors d'une étape suivante, on applique une déformation à la cellule. Pour cela on active les actionneurs en appliquant une tension d'actionnement continue aux bornes des électrodes de l'actionneur. L'actionneur engendre la déflexion voulue du support, appliquant une déformation à la cellule qui a adhéré sur le support. Cette déformation provoque l'apparition d'au moins un pore transitoire. La durée d'application de la contrainte à la cellule est de l'ordre de quelques secondes.

Le choix de la tension à appliquer aux actionneurs est fait par l'opérateur au moyen par exemple d'abaques donnant la déflexion du support de traitement en fonction de la tension appliquée pour réaliser un ou des pores transitoires dans des types donnés de cellules qui auront été réalisés lors de la fabrication du dispositif.

Lors d'une étape suivante, l'actionneur est désactivé en annulant la tension continue appliquée aux électrodes de l'actionneur, le support de traitement reprend sa position au repos et ramenant la cellule adhérente à un état non contraint, la membrane de la cellule comporte au moins un pore.

Tant que le ou les pores sont ouverts, les éléments à introduire contenus dans le milieu liquide pénètrent dans les cellules par le ou les pores.

Lors d'une étape suivante, aucune contrainte n'est appliquée à la cellule pour permettre la fermeture du ou des pores. Cette étape peut durer quelques minutes.

A la fin de l'étape de fermeture des pores, la cellule traitée est récupérée. On peut par exemple prévoir de mettre en vibration le support de traitement au moyen du ou des actionneurs et/ou d'appliquer un traitement enzymatique. Ces techniques de récupération permettent de ne pas endommager la cellule. Ensuite la cellule peut être récupérée dans le liquide en utilisant une pipette, ou par une circulation microfluidique transférant les cellules vers un réceptacle adapté. La cellule peut subir plusieurs traitements pour se voir délivrer différents types d'éléments.

Comme expliqué précédemment, ce dispositif de traitement présente l'avantage d'avoir un taux de mortalité des cellules très faible par rapport aux dispositifs de l'état de la technique.

Le dispositif de traitement peut comporter plusieurs supports de traitement distincts commandables de manière séparée ou commune.

La densité de cellules pouvant adhérer à un support dépend du type cellulaire mais à titre d'exemple, un ordre de grandeur de cette densité peut être de l'ordre de 100 000 cellules/cm².

Un exemple de procédé de réalisation d'un dispositif de traitement va maintenant être décrit, celui-ci comportant un support de traitement circulaire. Il s'agit avantageusement d'un procédé mettant en œuvre des techniques de la microélectronique, ce qui permet de réaliser un dispositif adapté à la taille de cellules. D'autres procédés pourraient être envisagés.

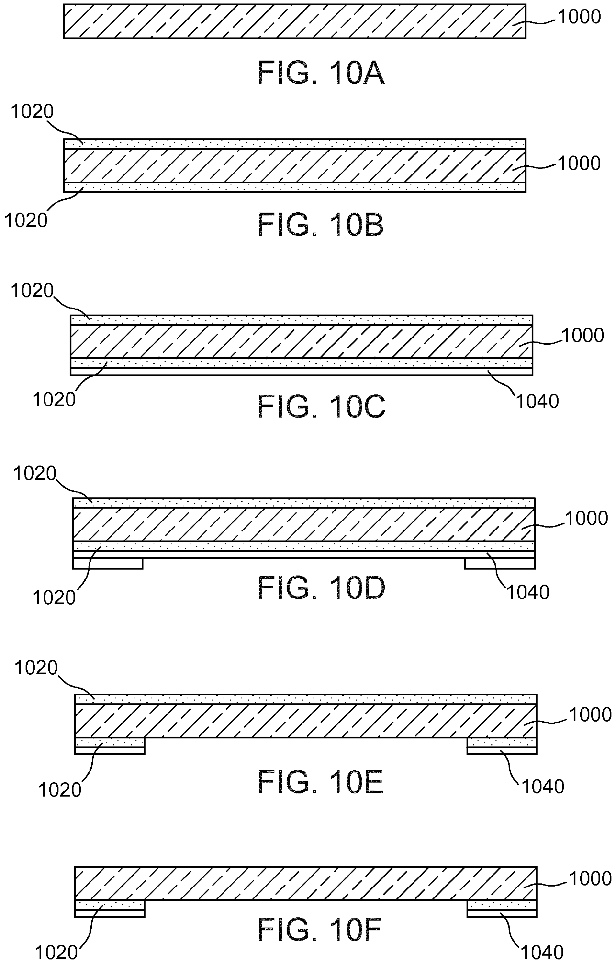

Les étapes sont représentées schématiquement sur les figures 10A à 10S.

Par exemple, on utilise un substrat en silicium 1000 représenté sur la figure 10A, ayant par exemple une épaisseur de 725 µm et un diamètre de 200mm de diamètre. Un substrat en verre par exemple peut être envisagé.

Lors d'une première étape, on effectue une oxydation thermique du substrat de sorte à former une couche d'oxyde 1020 sur toutes les surfaces du substrat d'une épaisseur de 2 µm par exemple. L'élément ainsi obtenu est représenté sur la figure 10B.

Ensuite, on réalise un masque dur d'oxyde 1040 sur la face arrière du substrat. Ce masque a par exemple une épaisseur de 7 µm. Le masque est réalisé en retournant le substrat; en fonction de la composition de dépôt choisie; il est possible de ne déposer le masque que sur cette face. Il peut s'agir par exemple d'un dépôt de type PVD (Physical Vapor Deposition en terminologie anglo-saxonne). L'élément ainsi obtenu est représenté sur la figure 10C.

On effectue ensuite une lithographie sur le masque dur. L'élément ainsi obtenu est représenté sur la figure 10D.

Lors d'une étape suivante, on grave, par exemple par gravure ionique réactive (RIE : Reactive-Ion Etching en terminologie anglo-saxonne), le masque dur et la couche d'oxyde 1020 en face arrière de sorte à atteindre la face arrière du substrat 1000. L'élément ainsi obtenu est représenté sur la figure 10E.

Lors d'une étape suivante, on retire la couche d'oxyde sur la face avant, par exemple par désoxydation ou gravure chimique. L'élément ainsi obtenu est représenté sur la figure 10F.

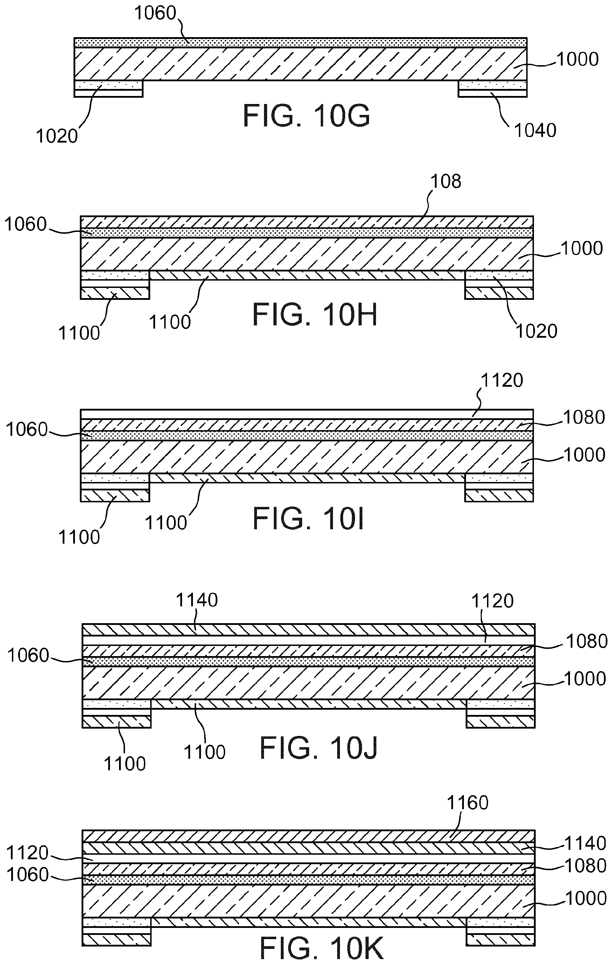

Lors d'une étape suivante, on forme une couche d'oxyde 1060 en face avant. Avantageusement, un recuit de densification a lieu par exemple à une température de l'ordre de 800°C. L'élément ainsi obtenu est représenté sur la figure 10G.

Lors d'une étape suivante, on forme une couche 1080 en face avant destinée à former la membrane 2, et une couche 1100 en face arrière. De préférence, ces couches sont par exemple en polysilicium, en SiC ou en SiO2. L'épaisseur des couches 1080, 1100 est par exemple comprise entre quelques centaines de nm à plusieurs µm, voire plusieurs dizaines de µm.

Les couches 1080, 1100 sont par exemple réalisées par dépôt chimique en phase vapeur (ou CVD pour Chemical Vapor Deposition en terminologie anglo-saxonne) ou par croissance épitaxiale. De préférence, les contraintes des couches 1080, 1100 sont maîtrisées.

Les couches 1080, 1100 peuvent être formées en plusieurs fois. Par exemple, pour une épaisseur de 4 µm, deux couches de 1,5 µm d'épaisseur et 1 couche de 1 µm d'épaisseur sont réalisées successivement.

Avantageusement une étape de recuit a ensuite lieu. L'élément ainsi obtenu est représenté sur la figure 10H.

Lors d'une étape suivante, une couche 1120 est formée sur la couche 1080 par exemple en SiO2 ou en SiN, ayant par exemple une épaisseur comprise entre quelques centaines de nm et plusieurs µm. La couche 1120 est formée par exemple par oxydation thermique ou par dépôt CVD. Avantageusement, un recuit de densification a lieu par exemple à une température de l'ordre de 800°C.

L'élément ainsi obtenu est représenté sur la figure 10I.

Lors d'une étape suivante, on réalise les premier et deuxième actionneurs.

Pour cela on réalise tout d'abord une couche 1140 destinée à former les électrodes inférieures des actionneurs, par exemple en Pt ou en Mo. La couche 1140 est réalisée par exemple par dépôt sur la couche 1120. La couche 1140 a par exemple une épaisseur comprise entre quelques dizaines de nm à quelques centaines de nm. L'élément ainsi obtenu est représenté sur la figure 10J.

Une couche de matériau piézoélectrique ou ferroélectrique 1160 est ensuite formée sur la couche 1140, par exemple en PZT, AIN, ZnO, LNO dont l'épaisseur est par exemple comprise entre quelques centaines de nm à quelques µm.

On réalise ensuite l'électrode supérieure par formation d'une couche 1180 sur le matériau piézoélectrique ou ferroélectrique 1160, par exemple en Ru ou en Au par exemple d'épaisseur comprise entre quelques dizaines de nm à quelques centaines de nm. L'élément ainsi obtenu est représenté sur la figure 10K.

Ont lieu ensuite des étapes de gravure.

Tout d'abord, la couche 1180 est gravée de sorte à délimiter l'actionneur annulaire 16 et l'actionneur en forme de disque 14.

Ensuite, la couche 1160 en matériau piézoélectrique ou ferroélectrique est gravée.

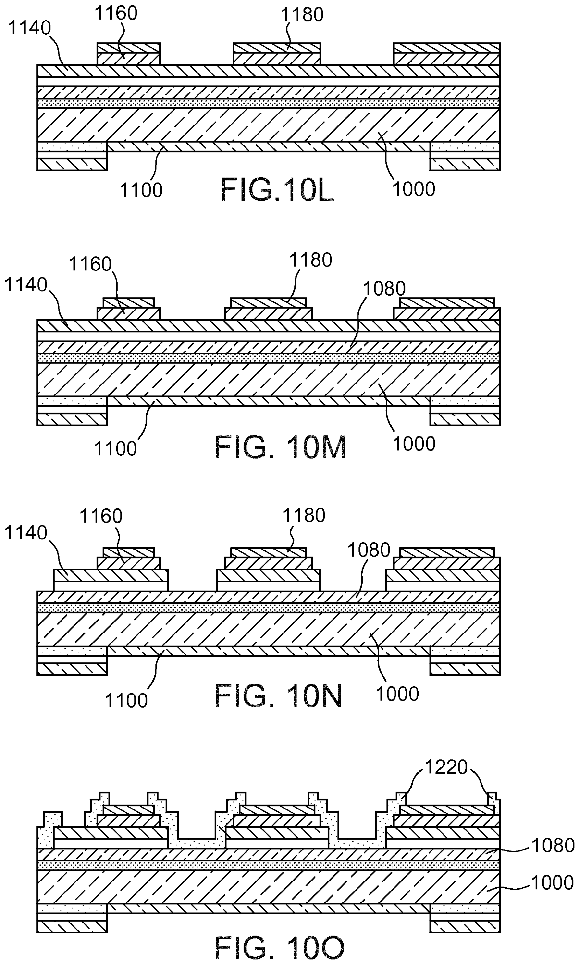

L'élément ainsi obtenu est représenté sur la figure 10L.

Ensuite, on grave à nouveau les portions de couche 1180 restantes de sorte à ce qu'elles soient en retrait par rapport aux portions de couche 1160. L'élément ainsi obtenu est représenté sur la figure 10M.

La couche 1140 est ensuite gravée, ainsi que la couche d'oxyde 1120.

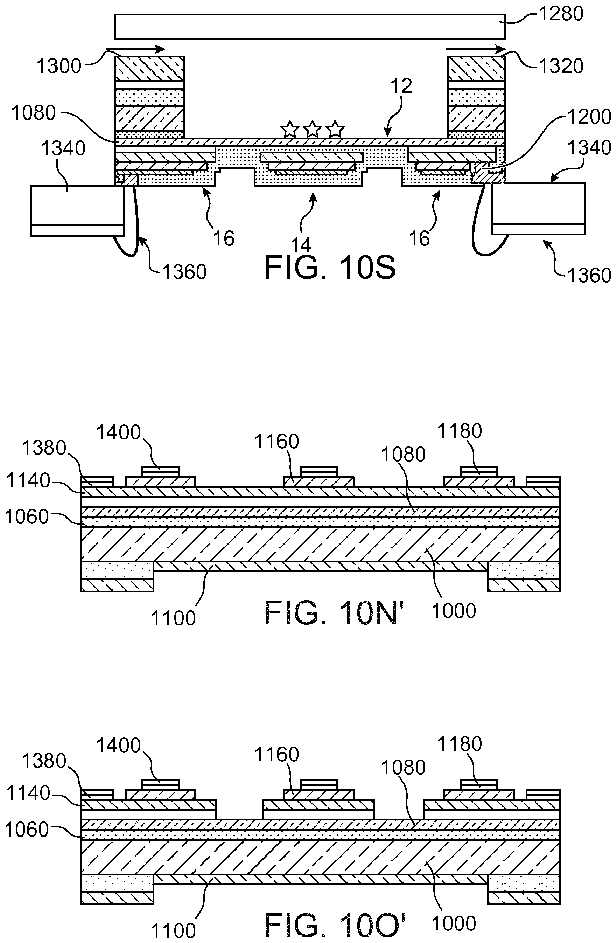

De préférence, on réalise un profil en escalier. Celui-ci est obtenu car toutes les couches sont déposées puis gravées, à partir de la couche supérieure, en utilisant des masques de photolithographie différents, le deuxième masque étant plus large que le premier, etc. Cela permet de laisser des marges de sécurité pour éviter le recouvrement de couches, qui pourrait apparaître du fait de l'incertitude de positionnement des masques. On évite ainsi tout court-circuit électrique entre les électrodes. L'élément ainsi obtenu est représenté sur la figure 10N.

Lors des étapes suivantes, on réalise des plots de reprise de contact 1200. Préalablement on forme une couche 1220 de matériau diélectrique, par exemple en SiO₂ sur les bords des empilements formés des électrodes inférieure, supérieure et du matériau piézoélectrique, cette couche étant gravée de sorte à dégager partiellement les électrodes inférieure et supérieure. L'élément ainsi obtenu est représenté sur la figure 10O.

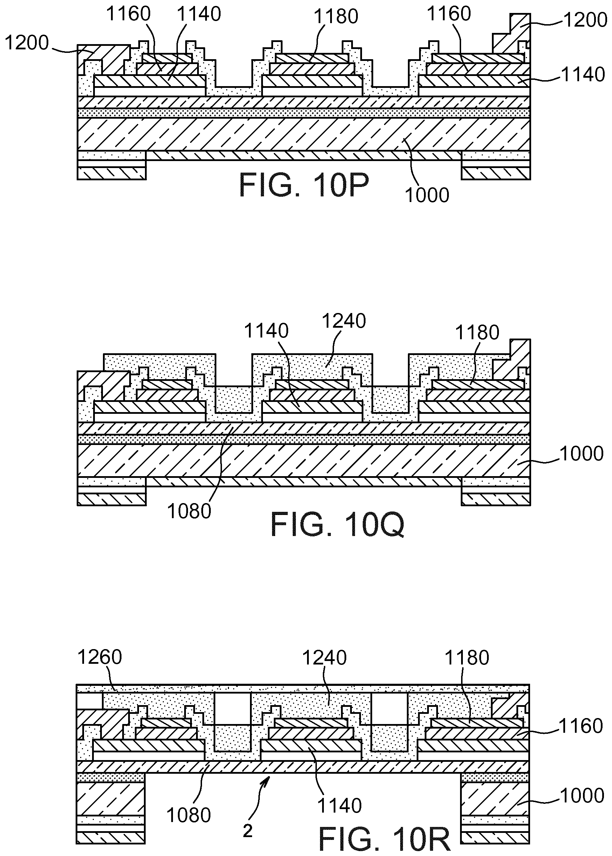

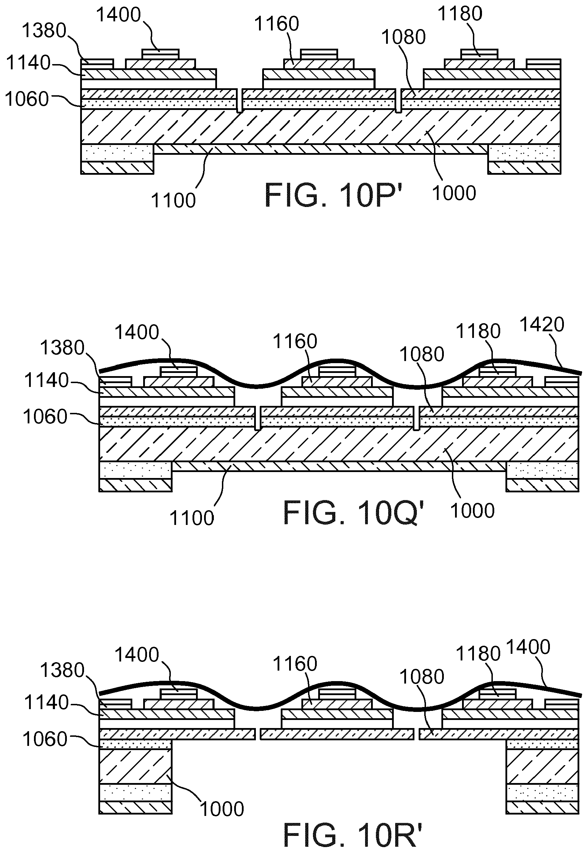

Ensuite, une couche par exemple en AISi ou en TiAu est formée et est gravée, formant ainsi des plots de contact au niveau des zones où les électrodes ont été dégagées. L'élément ainsi obtenu est représenté sur la figure 10P.

Avantageusement, lors d'une étape suivante on forme une couche de protection 1240 sur les actionneurs, par exemple une couche d'oxyde, afin de protéger les actionneurs du contact avec les éléments d'arrêt. L'épaisseur de cette couche peut être comprise entre quelques centaines de nm à quelques µm, par exemple 500nm.

Lors d'une étape suivante la couche 1240 est gravée, pour accéder aux reprises de contact.

L'élément ainsi obtenu est représenté sur la figure 10Q.

De préférence, lors d'une étape suivante, on protège les actionneurs, par exemple par le dépôt d'un film sec 1260. Ensuite, on grave la face arrière afin de libérer la membrane 2.

On libère la membrane par gravure profonde du substrat par la face arrière jusqu'à atteindre la membrane.

L'élément ainsi obtenu est représenté sur la figure 10R.

Pour pouvoir être utilisé en milieu liquide, un packaging adapté est réalisé. On ferme la cavité formée du côté de la face arrière de la membrane par un capot 1280. En effet on utilise préférentiellement la face arrière de la membrane pour effectuer le tri des cellules, ce qui simplifie les connexions électriques des actionneurs situés sur la face avant. Néanmoins les actionneurs étant encapsulés, la face avant pourvue des actionneurs peut également servir pour le tri en adaptant les connexions électriques. En utilisant à la fois la face arrière et la face avant, on double la capacité de tri du dispositif. Les deux faces de la membrane peuvent présenter les mêmes propriétés d'adhérence par rapport aux différents types de cellules ou des propriétés différentes, pour cela on peut fonctionnaliser l'une des faces.

Le capot 120 est par exemple en verre et est par exemple collé sur la couche 1100. On prévoit que le capot comporte un orifice d'entrée 1300 et un orifice de sortie 1320 pour le liquide contenant les cellules

En outre, le dispositif est disposé sur des supports 1340 de sorte à suspendre la membrane et à permettre sa mise en vibration. Les supports peuvent être des supports mécaniques ou une partie d'une plaque de circuit électronique. De manière avantageuse, les supports sont également utilisés pour l'alimentation électrique au moyen de microconnexions électriques 1360. Les microconnexions électriques sont obtenues par exemple en tendant des microfils, par exemple en or, qui sont soudés sur le plot de contact d'un côté, et sur la carte électronique de l'autre.

L'élément ainsi obtenu est représenté sur la figure 10S. Les cellules sont symbolisées par des étoiles.

Le dispositif peut faire partie d'un microdispositif, par exemple il peut former une partie de la surface d'une chambre microfluidique dans le cas d'un dispositif de tri. Il peut faire partie d'un circuit microfluidique et assurer la protection de l'intérieur de certains des canaux.

Dans l'exemple représenté sur la figure 10S, le support vibrant forme le fond de la chambre mais ceci n'est en aucun cas limitatif il peut former une paroi latérale voir une paroi supérieure. Il sera vérifié alors que ces parois entrent effectivement en contact avec la solution comprenant les cellules.

Pour réaliser un dispositif selon les figures 5A à 9B, les étapes de réalisation représentées sur les figures 10A à 10Q sont similaires. A l'étape représentée sur la figure 10R, un film en matériau souple par exemple en matériau polymère est déposé en lieu et place du film sec. Ensuite on libère le support de traitement par gravure profonde du substrat par la face arrière jusqu'à atteindre le support de traitement.

Les dispositifs représentés sur les figures 5A à 7B peuvent être réalisés selon le procédé suivant.

Les étapes 10A à 10M sont similaires.

On forme une couche 1380 par exemple en Ruthénium et ensuite une couche 1400 par exemple en or. On réalise ensuite une gravure de sorte à définir des plots de contact ruthénium-or sur le matériau PZT et sur le bord de la couche 1140.

L'élément ainsi obtenu est représenté sur la figure 10N'.

La couche 1140 est ensuite gravée, ainsi que la couche d'oxyde 1120.

De préférence, on réalise un profil en escalier. Celui-ci est obtenu car toutes les couches sont déposées puis gravées, à partir de la couche supérieure, en utilisant des masques de photolithographie différents, le deuxième masque étant plus large que le premier, etc. Cela permet de laisser des marges de sécurité pour éviter le recouvrement de couches, qui pourrait apparaître du fait de l'incertitude de positionnement des masques. On évite ainsi tout court-circuit électrique entre les électrodes. L'élément ainsi obtenu est représenté sur la figure 10O'.

Lors d'une étape suivante, on grave les couches 1080, 1060 et sur une partie de son épaisseur la couche 1000 afin de définir les poutres. L'élément ainsi obtenu est représenté sur la figure 10P'.

Lors d'une étape suivante, on dépose un film sec 1420 en face avant de l'élément de la figure 10P'. Le film sec sert à protéger la face avant de l'élément lors de la gravure profonde qui va suive. Le film est ensuite avantageusement conservé pour former le support souple sur lequel les cellules vont adhérer. Ce support sera déformé par activation des actionneurs.

L'élément ainsi obtenu est représenté sur la figure 10Q'.

Lors d'une étape suivante, on grave la face arrière afin de libérer les poutres par gravure profonde. On grave le substrat 1000 et la couche d'oxyde 1060 jusqu'à atteindre les poutres.

L'élément ainsi obtenu est représenté sur la figure 10R'.

## Revendications

1. Dispositif microfluidique de délivrance à au moins une cellule biologique (C) comportant :
- au moins un support de traitement (12, 112, 212, 312, 412, 512) comprenant une surface d'accueil permettant l'adhérence de ladite au moins une cellule biologique,
- des moyens d'alimentation en une solution comportant au moins une cellule biologique à traiter,
- des moyens d'évacuation de ladite solution,
- des moyens pour alimenter en une solution contenant des éléments à introduire dans la cellule,
- des moyens configurés pour appliquer une contrainte à la cellule (C) pour provoquer l'apparition d'au moins un pore transitoire dans la membrane de la cellule (C) par déformation du support de traitement, lesdits moyens comportant au moins un actionneur (14, 16, 214, 314, 414, 514) apte à déformer ledit support afin d'appliquer une contrainte à ladite au moins une cellule, et des moyens de commande (UC) dudit au moins un actionneur de sorte que l'actionneur déforme le support de traitement (12, 112, 212, 312, 412, 512) selon une amplitude de déformation donnée et pendant une durée de traitement donnée de sorte à appliquer ladite contrainte à la cellule pendant ladite durée de traitement, ladite amplitude de déformation étant telle qu'elle génère au moins un pore transitoire dans une membrane de la cellule biologique (C) de sorte à traiter ladite cellule.

2. Dispositif microfluidique de délivrance selon la revendication 1, dans lequel ladite amplitude de déformation permet de déterminer la réponse mécanique de la cellule à la contrainte de sorte à la caractériser.

3. Dispositif microfluidique de délivrance selon la revendication 1 ou 2, dans lequel ledit support de traitement (12, 112, 212, 312, 412) est contenu principalement dans plan moyen (P) et dans lequel ledit actionneur (14, 16, 114, 214, 314, 414) est apte à déformer le support de traitement (12, 112, 212, 312, 412) selon une direction hors plan.

4. Dispositif microfluidique de délivrance selon la revendication 1 ou 2, dans lequel ledit support de traitement (512) est contenu principalement dans plan moyen (P) et dans lequel ledit actionneur (514) est apte à déformer le support de traitement (512) principalement dans ledit plan moyen.

5. Dispositif microfluidique de délivrance selon l'une quelconque des revendications 1 à 4, comportant un actionneur ou plusieurs actionneurs disposés de sorte à appliquer une déformation axisymétrique au support de traitement.

6. Dispositif microfluidique de délivrance selon l'une quelconque des revendications 1 à 4, comportant un actionneur ou plusieurs actionneurs (314, 414) disposés de sorte à appliquer une déformation cylindrique au support de traitement (312, 412).

7. Dispositif microfluidique de délivrance selon l'une quelconque des revendications 1 à 6, dans lequel tout ou partie de la surface du support de traitement (12, 112, 212, 312, 412, 512) est fonctionnalisée de sorte à modifier la force d'adhérence du ou des types de cellules biologiques à traiter par rapport à la surface non fonctionnalisée.

8. Dispositif microfluidique de délivrance selon l'une quelconque des revendications 1 à 7, dans lequel ledit actionneur (14, 16, 214, 314, 414) est un actionneur piézoélectrique ou ferroélectrique ou thermique.

9. Dispositif microfluidique de délivrance selon l'une quelconque des revendications 1 à 7, dans lequel ledit actionneur (114, 514) est un actionneur électrostatique ou magnétique

10. Dispositif microfluidique de délivrance selon l'une quelconque des revendications 1 à 9 en combinaison avec la revendication 4, comportant un support de traitement (512) en matériau souple et dans lequel ledit au moins actionneur (514) est tel qu'il peut appliquer un effort de traction à une zone de bord du support de traitement (512) dans ledit plan moyen (P), avantageusement ledit dispositif comportant au moins deux actionneurs (514) appliquant un effort de traction en sens opposés à deux bords opposés du support de traitement (512), de manière avantageuse les actionneurs (514) sont des actionneurs électrostatiques à peignes interdigités, chaque actionneur comportant un peigne mobile (518) et un peigne fixe (530) définissant entre eux un entrefer, et dans lequel le support de traitement (512) est suspendu entre les deux peignes mobiles (528), la variation, avantageusement la réduction, dudit entrefer appliquant une contrainte de traction audit support de traitement (512).

11. Dispositif microfluidique de délivrance selon les revendications 3 et 8, dans lequel le support de traitement est en un matériau souple (212, 312, 412) et l'actionneur (214, 314, 414) comporte au moins une poutre encastrée-libre (226, 326) et un matériau piézoélectrique ou ferroélectrique (224, 324) solidaire de la poutre(226, 326), de sorte que la déformation du matériau piézoélectrique ou ferroélectrique (224, 324) provoque une déformation hors plan de la poutre (226, 326) et du support de traitement (214, 314, 414), ledit dispositif comportant avantageusement plusieurs actionneurs répartis au niveau d'un bord extérieur du support de traitement.

12. Dispositif microfluidique de délivrance selon l'une quelconque des revendications 1 à 11, étant un dispositif MEMS et/ou NEMS

13. Procédé de délivrance cellulaire mettant en œuvre le dispositif microfluidique de délivrance cellulaire selon l'une des revendications 1 à 12, comportant les étapes:
- mise en contact de la solution contenant la au moins une cellule biologique (C) à traiter avec le support de traitement (12, 112, 212, 312, 412, 512),
- adhérence de ladite au moins une cellule (C) au support de traitement (12, 112, 212, 312, 412, 512),
- alimentation en solution contenant les éléments à introduire dans la cellule,
- application d'une contrainte à la cellule (C) pour provoquer l'apparition d'au moins un pore transitoire dans la membrane de la cellule (C) par déformation du support de traitement (12, 112, 212, 312, 412, 512),
- arrêt de l'application de la contrainte,
- fermeture du au moins un pore transitoire,
- évacuation de la cellule (C).

14. Procédé de délivrance cellulaire selon la revendication 13, dans lequel le support de traitement(512) est déformé dans son plan ou le support de traitement (12, 112, 212, 312, 412) est déformé selon une direction hors plan.

## Patentansprüche

1. Mikrofluidische Vorrichtung zur Abgabe an mindestens eine biologische Zelle (C), enthaltend:
- zumindest eine Prozesshalterung (12, 112, 212, 312, 412, 512), die eine Aufnahmefläche aufweist, die das Anhaften der zumindest einen biologischen Zelle ermöglicht,
- Mittel zum Zuführen einer Lösung, die zumindest eine zu behandelnde biologische Zelle enthält,
- Mittel zum Abführen der Lösung,
- Mittel zum Zuführen einer Lösung, die Elemente enthält, die in die Zelle eingeführt werden sollen,
- Mittel, die dazu ausgelegt sind, um eine Spannung auf die Zelle (C) auszuüben, um zu bewirken, dass zumindest eine transiente Pore in der Zellmembran der Zelle (C) durch Verformung der Prozesshalterung erscheint, wobei die Mittel zumindest ein Betätigungsglied (14, 16, 214, 314, 414, 514) enthalten, das dazu geeignet ist, die Halterung zu verformen, um eine Spannung auf die zumindest eine Zelle auszuüben, sowie Steuermittel (UC) zum Steuern des zumindest einen Betätigungsglieds, so dass das Betätigungsglied die Prozesshalterung (12, 112, 212, 312, 412, 512) gemäß einer gegebenen Verformungsamplitude und während einer gegebenen Prozesszeit verformt, um die Spannung während der Prozesszeit auf die Zelle auszuüben, wobei die Verformungsamplitude derart ist, dass sie zumindest eine transiente Pore in einer Membran der biologischen Zelle erzeugt (C), um die Zelle zu behandeln.

2. Mikrofluidische Abgabevorrichtung nach Anspruch 1,
wobei
die Verformungsamplitude ermöglicht, die mechanische Reaktion der Zelle auf die Spannung zu bestimmen, um sie zu charakterisieren.

3. Mikrofluidische Abgabevorrichtung nach Anspruch 1 oder 2,
wobei
die Prozesshalterung (12, 112, 212, 312, 412) im Wesentlich in einer mittleren Ebene (P) enthalten ist und
das Betätigungsglied (14, 16, 114, 214, 314, 414) dazu geeignet ist, die Prozesshalterung (12, 112, 212, 312, 412) in einer Richtung außerhalb der Ebene zu verformen.

4. Mikrofluidische Abgabevorrichtung nach Anspruch 1 oder 2,
wobei
die Prozesshalterung (512) im Wesentlich in einer mittleren Ebene (P) enthalten ist und
das Betätigungsglied (514) dazu geeignet ist, die Prozesshalterung (512) im Wesentlichen in der mittleren Ebene zu verformen.

5. Mikrofluidische Abgabevorrichtung nach einem der Ansprüche 1 bis 4, enthaltend ein oder mehrere Betätigungsglieder, die so angeordnet sind, dass sie eine achsensymmetrische Verformung auf die Prozesshalterung ausüben.

6. Mikrofluidische Abgabevorrichtung nach einem der Ansprüche 1 bis 4, enthaltend ein oder mehrere Betätigungsglieder (314, 414), die so angeordnet sind, dass sie eine zylindrische Verformung auf die Prozesshalterung (312, 412) ausüben.

7. Mikrofluidische Abgabevorrichtung nach einem der Ansprüche 1 bis 6,
wobei
die gesamte oder ein Teil der Oberfläche der Prozesshalterung (12, 112, 212, 312, 412, 512) so funktionalisiert, dass die Anhaftkraft der zu behandelnden Art(en) biologischer Zellen in Bezug auf die nichtfunktionalisierte Oberfläche verändert wird.

8. Mikrofluidische Abgabevorrichtung nach einem der Ansprüche 1 bis 7,
wobei
das Betätigungsglied (14, 16, 214, 314, 414) ein piezoelektrisches oder ferroelektrisches oder thermisches Betätigungsglied ist.

9. Mikrofluidische Abgabevorrichtung nach einem der Ansprüche 1 bis 7,
wobei
das Betätigungsglied (114, 514) ein elektrostatisches oder magnetisches Betätigungsglied ist.

10. Mikrofluidische Abgabevorrichtung nach einem der Ansprüche 1 bis 9 in Kombination mit Anspruch 4,
enthaltend eine Prozesshalterung (512) aus nachgiebigem Material, wobei das zumindest eine Betätigungsglied (514) derart ist, dass es eine Zugkraft auf einen Randbereich der Prozesshalterung (512) in der mittleren Ebene (P) ausüben kann, wobei die Vorrichtung vorteilhaft zumindest zwei Betätigungsglieder (514) enthält, die eine Zugkraft in entgegengesetzten Richtungen auf zwei entgegengesetzte Ränder der Prozesshalterung (512) ausüben, wobei vorteilhaft die Betätigungsglieder (514) elektrostatische Betätigungsglieder mit interdigitalen Kämmen sind, wobei jedes Betätigungsglied einen beweglichen Kamm (518) und einen festen Kamm (530) enthält, die zwischen sich einen Spalt definieren, und wobei die Prozesshalterung (512) zwischen den beiden beweglichen Kämmen (528) abgehängt ist, wobei durch die Veränderung, vorteilhaft durch die Verminderung, des Spaltes eine Zugspannung auf die Prozesshalterung (512) ausgeübt wird.

11. Mikrofluidische Abgabevorrichtung nach den Ansprüchen 3 und 8,
wobei die Prozesshalterung aus nachgiebigem Material (212, 312, 412) besteht und das Betätigungsglied (214, 314, 414) zumindest einen eingelassenen, freien Träger (226, 326) und ein mit dem Träger (226, 326) fest verbundenes piezoelektrisches oder ferroelektrisches Material (224, 324) enthält, so dass die Verformung des piezoelektrischen oder ferroelektrischen Materials (224, 324) eine Verformung des Trägers (226, 326) und der Prozesshalterung (214, 314, 414) nach außerhalb der Ebene bewirkt, wobei die Vorrichtung vorzugsweise mehrere Betätigungsglieder aufweist, die im Bereich eines äußeren Randes der Prozesshalterung verteilt sind.

12. Mikrofluidische Abgabevorrichtung nach einem der Ansprüche 1 bis 11,
wobei
sie eine MEMS- und/oder NEMS-Vorrichtung ist.

13. Verfahren zur Zellenabgabe unter Verwendung der mikrofluidischen Vorrichtung zur Zellenabgabe nach einem der Ansprüche 1 bis 12, umfassend die Schritte:
- Inkontaktbringen der Lösung, die die zumindest eine zu behandelnde biologische Zelle (C) enthält, mit der Prozesshalterung (12, 112, 212, 312, 412, 512),
- Anhaften der zumindest einen Zelle (C) an die Prozesshalterung (12, 112, 212, 312, 412, 512),
- Zuführen von Lösung, die die Elemente enthält, die in die Zelle eingeführt werden sollen,
- Ausüben einer Spannung auf die Zelle (C), um zu bewirken, dass zumindest eine transiente Pore in der Zellmembran der Zelle (C) durch Verformung der Prozesshalterung (12, 112, 212, 312, 412, 512) erscheint,
- Stoppen der Spannungsausübung,
- Schließen der zumindest einen transienten Pore,
- Abführen der Zelle (C).

14. Verfahren zur Zellenabgabe nach Anspruch 13,
wobei
die Prozesshalterung (512) in ihrer Ebene verformt wird oder die Prozesshalterung (12, 112, 212, 312, 412) in einer Richtung außerhalb der Ebene verformt wird.

## Claims

1. Microfluidic device for delivery to at least one cell comprising :
at least one treatment support (12, 112, 212, 312, 412, 512) comprising a receiving surface enabling the adhesion of said at least one biological cell,
means for supplying a solution comprising at least one biological cell to treat,
means for evacuating said solutions
- means configured to apply a stress to the cell (C) to provoke deform said treatment support to generate at least one transitory pore in a membrane of the cell by deforming the treatment support, said means comprising at least one actuator (14, 16, 214, 314, 414, 514) able to deform said support in order to apply a stress to said at least one cell, and means (UC) for controlling said at least one actuator such that the actuator deforms the treatment support (12, 112, 212, 312, 412, 512) according to a given amplitude of deformation and for a given treatment duration so as to apply said stress to the cell for said treatment duration, said amplitude of deformation is such that it generates at least one transitory pore in a membrane of the biological cell© so as to treat said cell.

2. Delivery microfluidic device according to claim 1, in which said given amplitude of deformation makes it possible to determine the mechanical response of the cell to the stress so as to characterise it.

3. Delivery microfluidic device according to claim 1 or 2, in which said treatment support (12, 112, 212, 312, 412, 512) is contained mainly in the median plane (P) and in which said actuator (14, 16, 114, 214, 314, 414, 514) is configured to deform the treatment support(12, 112, 212, 312, 412, 512) along an out-of-plane direction.

4. Delivery microfluidic device according to claim 1 or 2, in which said treatment support (512) is contained mainly in the median plane (P) and in which said actuator (514) is configured to deform the treatment support (512)mainly in said median plane.

5. Delivery microfluidic device according to one of the claims 1 to 4, comprising one actuator or several actuators arranged so as to apply an axisymmetric deformation to the treatment support.

6. Delivery microfluidic device according to one of the claims 1 to 4, comprising one actuator or several actuators (314, 414) arranged so as to apply a cylindrical deformation to the treatment support (312, 412).

7. Delivery microfluidic device according to one of the claims 1 to 6, in which all or part of the surface of the treatment support (12, 112, 212, 312, 412, 512) is functionalised so as to modify the adhesion force of the type or types of biological cells to treat with respect to the non-functionalised surface.

8. Delivery microfluidic device according to one of the claims 1 to 7, in which said actuator (14, 16, 214, 314, 414) is a piezoelectric or ferroelectric or thermal actuator.

9. Delivery microfluidic device according to one of the claims 1 to 7, in which said actuator (114, 514) is an electrostatic or magnetic actuator.

10. Delivery microfluidic device according to one of the claims 1 to 9 in combination with claim 4, in which the treatment support (512) is made of a flexible material and in which the at least one actuator (514) is such that it can apply a tensile force to an edge area of the treatment support (512) in said median plane (P), advantageously said device comprising at least two actuators (514) applying a tensile force in opposite directions to two opposite edges of the treatment support (512), in advantageous manner, the actuators (514) are electrostatic actuators with interdigitated combs, each actuator comprising a moveable comb (518) and a fixed comb (530) defining between them an air gap, and in which the treatment support (512) is suspended between the two moveable combs (528), the variation, advantageously the reduction, of said air gap applying a tensile stress to said treatment support (512)..

11. Delivery microfluidic device according to claims 3 and 8, in which the support treatment is made of flexible material (212, 312, 412) and the actuator (214, 314, 414) comprises at least one fixed-free beam (226, 326) and a piezoelectric or ferroelectric material (224, 324) secured to the beam (226, 326), such that the deformation of the piezoelectric or ferroelectric material (224, 324) causes an out-of-plane deformation of the beam (226, 326) and the treatment support (214, 314, 414), said device advantageously comprising several actuators spread out at the level of an outer edge of the treatment support.

12. Delivery microfluidic device according to one of the claims 1 to 11, in which the device is MEMS and/or NEMS device.

13. Cellular delivery method using a Cellular delivery microfluidic device according to one of the claims 1 to 12 comprising steps:
- placing the solution containing at least one cell (C) to treat in contact with a treatment support (12, 112, 212, 312, 412, 512),
- adhesion of said at least one cell (C) to the treatment support (12, 112, 212, 312, 412, 512),
- supplying a solution containing elements to introduce into the cell,
- applying a stress to the cell (C) to cause the appearance of at least one transitory pore in a membrane of the cell (C) by deformation of the treatment support (12, 112, 212, 312, 412, 512),
- stopping the application of the stress,
- closing the at least one transitory pore,
- evacuating the cell (C).

14. Method of cellular delivery according to claim 13, in which the treatment support (512) is deformed in its plane or the treatment support (12, 112, 212, 312, 412 is deformed along an out-of-plane direction.
